# EUROPEAN PATENT APPLICATION

(11) **EP 4 550 068 A1**
(43) Date of publication of application: **07.05.2025**
(21) Application number: 23207978.0
(22) Date of filing: 06.11.2023
(51) Int. Cl.: G05B 19/418, G06Q 10/087, G16H 40/20

(54) **SYSTEM, METHOD, AND COMPUTER PROGRAM PRODUCT FOR SINGULATED MEDICAL DEVICES ON HIGH-THROUGHPUT MACHINES**

(71) Applicant: Becton, Dickinson and Company, Franklin Lakes, NJ 07147-1880 (US)
(72) Inventor: RIVIER, Cédric, 38340 VOREPPE (FR); MESSAOUD, Mourad, 38000 Grenoble (FR); PERRODIN, Jérémie, 38100 Grenoble (FR); GAGET, Laurent, 38180 Seyssins (FR)
(74) Representative: Germain Maureau

(57) **Abstract**

A method may include reading, with a radio-frequency identification (RFID) reader, from a plurality of RFID tags on a plurality of medical devices in a production line, a plurality of identifiers associated with the plurality of medical devices, the plurality of medical devices in the production line being individually moved adjacent to an antenna of the RFID reader by a conveyor. For each identifier of the plurality of identifiers, at least one processor may determine whether that identifier is included in a list of expected identifiers, and in response to determining that that identifier is not included in the list of expected identifiers, control the conveyor to remove a medical device of the plurality of medical devices associated with that identifier from the production line.

## Description

### BACKGROUND

### 1. Field

The present disclosure relates generally to high-throughput production and, in some non-limiting embodiments or aspects, to systems, methods, and computer program products for anti-mix up of singulated syringes on high-throughput production machines.

### 2. Technical Considerations

Existing techniques for avoiding product mix-ups in production lines may use colored identifiers (e.g., colored rings, etc.) placed on products after a visual inspection process. Before a stage in which the colored rings are placed on the products, anti-mix up techniques may typically rely on manual inspection processes. Moreover, a color panel used for anti-mix up identifiers may be limited.

### SUMMARY

Accordingly, provided are improved systems, devices, products, apparatus, and/or methods for singulated medical devices on high-throughput machines.

According to some non-limiting embodiments or aspects, provided is a system, including: a radio-frequency identification (RFID) reader configured to read, from a plurality of RFID tags on a plurality of medical devices in a production line, a plurality of identifiers associated with the plurality of medical devices; a conveyor configured to individually move the plurality of medical devices in the production line adjacent to an antenna of the RFID reader; and at least one processor coupled to a memory and configured to: for each identifier of the plurality of identifiers, determine whether that identifier is included in a list of expected identifiers; and in response to determining that that identifier is not included in the list of expected identifiers, control the conveyor to remove a medical device of the plurality of medical devices associated with that identifier from the production line.

In some non-limiting embodiments or aspects, the system further includes: at least one sensor configured to detect when an individual medical device is in a predetermined location relative to the antenna of the RFID reader, wherein, for each medical device of the plurality of medical devices in the production line: the at least one sensor is configured to transmit a trigger signal to the RFID reader to cause the RFID reader to attempt to read a RFID tag of the plurality of RFID tags on that medical device in response to detecting that that medical device is in the predetermined location relative to the antenna of the RFID reader in the production line; and the RFID reader is configured to attempt to read the RFID tag on that medical device in response to receiving the trigger signal from the at least one sensor.

In some non-limiting embodiments or aspects, in response to at least one attempt to read at least one RFID tag on at least one medical device that fails to read at least one identifier associated with the at least one medical device from the at least one RFID tag, the at least one processor is further configured to control the conveyor to remove the at least one medical device from the production line.

In some non-limiting embodiments or aspects, the system further includes: a local database configured to store the list of expected identifiers, wherein, for each identifier, the at least one processor is configured to determine whether that identifier is included in the list of expected identifiers by querying, based on that identifier, the local database including the list of expected identifiers.

In some non-limiting embodiments or aspects, for each identifier, the at least one processor is configured to immediately query, based on that identifier, the local database including the list of expected identifiers in response to reading, with the RFID reader, that identifier.

In some non-limiting embodiments or aspects, the conveyor is configured to individually move the plurality of medical devices in the production line adjacent to the antenna of the RFID reader in a series of cycles, wherein each cycle of the series of cycles includes a subset of medical devices of the plurality of medical devices in the production line being individually moved adjacent to the antenna of the RFID reader by the conveyor, and wherein the at least one processor is further configured to: for each cycle of the series of cycles: after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.

In some non-limiting embodiments or aspects, the at least one processor is further configured to: obtain a batch number associated with the plurality of medical devices; and control, based on the batch number, the local database to synchronize the list of expected identifiers with an external database that includes a plurality of lists of expected identifiers associated with a plurality of batch numbers.

In some non-limiting embodiments or aspects, the plurality of medical devices includes a plurality of syringes.

In some non-limiting embodiments or aspects, the plurality of identifiers includes a plurality of electronic product codes.

In some non-limiting embodiments or aspects, the at least one processor is further configured to: for each identifier of the plurality of identifiers: in response to determining that that identifier is included in the list of expected identifiers, control the conveyor to maintain the medical device of the plurality of medical devices associated with that identifier in the production line.

According to some non-limiting embodiments or aspects, provided is a method, including: reading, with a radio-frequency identification (RFID) reader, from a plurality of RFID tags on a plurality of medical devices in a production line, a plurality of identifiers associated with the plurality of medical devices, wherein the plurality of medical devices in the production line are individually moved adjacent to an antenna of the RFID reader by a conveyor; and for each identifier of the plurality of identifiers: determining, with at least one processor, whether that identifier is included in a list of expected identifiers; and in response to determining that that identifier is not included in the list of expected identifiers, controlling, with the at least one processor, the conveyor to remove a medical device of the plurality of medical devices associated with that identifier from the production line.

In some non-limiting embodiments or aspects, the method further includes: for each medical device of the plurality of medical devices in the production line: detecting, with at least one sensor, when that medical device is in a predetermined location relative to the antenna of the RFID reader; in response to detecting, with the at least one sensor, that that medical device is in the predetermined location relative to the antenna of the RFID reader in the production line, transmitting, with the at least one sensor, a trigger signal to the RFID reader to trigger the RFID reader to attempt to read a RFID tag of the plurality of RFID tags on that medical device; and in response to receiving the trigger signal from the at least one sensor, attempting to read, with the RFID reader, the RFID tag on that medical device.

In some non-limiting embodiments or aspects, the method further includes: in response to at least one attempt to read at least one RFID tag on at least one medical device that fails to read at least one identifier associated with the at least one medical device from the at least one RFID tag, controlling, with the at least one processor, the conveyor to remove the at least one medical device from the production line.

In some non-limiting embodiments or aspects, for each identifier, determining, with the at least one processor, whether that identifier is included in the list of expected identifiers, includes: querying, with the at least one processor, based on that identifier, a database including the list of expected identifiers.

In some non-limiting embodiments or aspects, for each identifier, the database including the list of expected identifiers is immediately queried, based on that identifier, in response to reading, with the RFID reader, that identifier.

In some non-limiting embodiments or aspects, the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the method further comprises: for each cycle of the series of cycles: after each medical device included in the subset of medical devices in that cycle is individually moved adjacent to the antenna of the RFID reader by the conveyor, querying, with the at least one processor, based on a subset of identifiers associated with that subset of medical devices in that cycle, the database including the list of expected identifiers.

In some non-limiting embodiments or aspects, the method further includes: obtaining, with the at least one processor, a batch number associated with the plurality of medical devices; and controlling, with the at least one processor, based on the batch number, the database to synchronize the list of expected identifiers with a further database that includes a plurality of lists of expected identifiers associated with a plurality of batch numbers.

In some non-limiting embodiments or aspects, the plurality of medical devices includes a plurality of syringes.

In some non-limiting embodiments or aspects, the plurality of identifiers includes a plurality of electronic product codes.

In some non-limiting embodiments or aspects, the method further includes: for each identifier of the plurality of identifiers: in response to determining that that identifier is included in the list of expected identifiers, controlling, with the at least one processor, the conveyor to maintain the medical device of the plurality of medical devices associated with that identifier in the production line.

According to some non-limiting embodiments or aspects, provided is a computer program product including at least one non-transitory computer-readable medium including program instructions that, when executed by at least one processor, cause the at least one processor to: receive, from a radio-frequency identification (RFID) reader, a plurality of identifiers read from a plurality of RFID tags on a plurality of medical devices in production line, wherein the plurality of medical devices in the production line are individually moved adjacent to an antenna of the RFID reader by a conveyor; and for each identifier of the plurality of identifiers: determine whether that identifier is included in a list of expected identifiers; and in response to determining that that identifier is not included in the list of expected identifiers, transmit, to the conveyor, a signal to remove a medical device of the plurality of medical devices associated with that identifier from the production line.

In some non-limiting embodiments or aspects, for each medical device of the plurality of medical devices in the production line: at least one sensor detects when that medical device is in a predetermined location relative to the antenna of the RFID reader and transmits, in response to detecting that that medical device is in the predetermined location relative to the antenna of the RFID reader, a trigger signal to the RFID reader to trigger the RFID reader to attempt to read a RFID tag of the plurality of RFID tags on that medical device; and the RFID reader attempts to read the RFID tag on that medical device in response to receiving the trigger signal from the at least one sensor, and wherein the program instructions, when executed by the at least one processor, further cause the at least one processor to: for each medical device of the plurality of medical devices in the production line, receive, from the RFID reader, at least one of: (i) an indication of whether a read attempt associated with that medical device failed to read an identifier associated with that medical device, (ii) the identifier associated with that medical device, or any combination thereof.

In some non-limiting embodiments or aspects, the program instructions, when executed by the at least one processor, further cause the at least one processor to: in response to receiving an indication that the read attempt associated with that medical device failed, control the conveyor to remove that medical device from the production line.

In some non-limiting embodiments or aspects, the program instructions, when executed by the at least one processor, further cause the at least one processor to determine, for each identifier, whether that identifier is included in the list of expected identifiers by: querying, based on that identifier, a database including the list of expected identifiers.

In some non-limiting embodiments or aspects, the program instructions, when executed by the at least one processor, cause the at least one processor to immediately query, for each identifier, the database including the list of expected identifiers in response to receiving that identifier from the RFID reader.

In some non-limiting embodiments or aspects, the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the program instructions, when executed by the at least one processor, further cause the at least one processor to: for each cycle of the series of cycles: after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.

In some non-limiting embodiments or aspects, the program instructions, when executed by the at least one processor, further cause the at least one processor to: obtain a batch number associated with the plurality of medical devices; and control, based on the batch number, the database to synchronize the list of expected identifiers with a further database that includes a plurality of lists of expected identifiers associated with a plurality of batch numbers.

In some non-limiting embodiments or aspects, the plurality of medical devices includes a plurality of syringes.

In some non-limiting embodiments or aspects, the plurality of identifiers includes a plurality of electronic product codes.

In some non-limiting embodiments or aspects, the program instructions, when executed by the at least one processor, further cause the at least one processor to: for each identifier of the plurality of identifiers: in response to determining that that identifier is included in the list of expected identifiers, control the conveyor to maintain the medical device of the plurality of medical devices associated with that identifier in the production line.

Further non-limiting embodiments or aspects are set forth in the following numbered clauses:
Clause 1. A system, comprising: a radio-frequency identification (RFID) reader configured to read, from a plurality of RFID tags on a plurality of medical devices in a production line, a plurality of identifiers associated with the plurality of medical devices; a conveyor configured to individually move the plurality of medical devices in the production line adjacent to an antenna of the RFID reader; and at least one processor coupled to a memory and configured to: for each identifier of the plurality of identifiers: determine whether that identifier is included in a list of expected identifiers; and in response to determining that that identifier is not included in the list of expected identifiers, control the conveyor to remove a medical device of the plurality of medical devices associated with that identifier from the production line.
Clause 2. The system of clause 1, further comprising: at least one sensor configured to detect when an individual medical device is in a predetermined location relative to the antenna of the RFID reader, wherein, for each medical device of the plurality of medical devices in the production line: the at least one sensor is configured to transmit a trigger signal to the RFID reader to cause the RFID reader to attempt to read a RFID tag of the plurality of RFID tags on that medical device in response to detecting that that medical device is in the predetermined location relative to the antenna of the RFID reader in the production line; and the RFID reader is configured to attempt to read the RFID tag on that medical device in response to receiving the trigger signal from the at least one sensor.
Clause 3. The system of clause 1 or 2, wherein, in response to at least one attempt to read at least one RFID tag on at least one medical device that fails to read at least one identifier associated with the at least one medical device from the at least one RFID tag, the at least one processor is further configured to control the conveyor to remove the at least one medical device from the production line.
Clause 4. The system of any of clauses 1-3, further comprising: a local database configured to store the list of expected identifiers, wherein, for each identifier, the at least one processor is configured to determine whether that identifier is included in the list of expected identifiers by querying, based on that identifier, the local database including the list of expected identifiers.
Clause 5. The system of any of clauses 1-4, wherein, for each identifier, the at least one processor is configured to immediately query, based on that identifier, the local database including the list of expected identifiers in response to reading, with the RFID reader, that identifier.
Clause 6. The system of any of clauses 1-5, wherein the conveyor is configured to individually move the plurality of medical devices in the production line adjacent to the antenna of the RFID reader in a series of cycles, wherein each cycle of the series of cycles includes a subset of medical devices of the plurality of medical devices in the production line being individually moved adjacent to the antenna of the RFID reader by the conveyor, and wherein the at least one processor is further configured to: for each cycle of the series of cycles: after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.
Clause 7. The system of any of clauses 1-6, wherein the at least one processor is further configured to: obtain a batch number associated with the plurality of medical devices; and control, based on the batch number, the local database to synchronize the list of expected identifiers with an external database that includes a plurality of lists of expected identifiers associated with a plurality of batch numbers.
Clause 8. The system of any of clauses 1-7, wherein the plurality of medical devices includes a plurality of syringes.
Clause 9. The system of any of clauses 1-8, wherein the plurality of identifiers includes a plurality of electronic product codes.
Clause 10. The system of any of clauses 1-9, wherein the at least one processor is further configured to: for each identifier of the plurality of identifiers: in response to determining that that identifier is included in the list of expected identifiers, control the conveyor to maintain the medical device of the plurality of medical devices associated with that identifier in the production line.
Clause 11. A method, comprising: reading, with a radio-frequency identification (RFID) reader, from a plurality of RFID tags on a plurality of medical devices in a production line, a plurality of identifiers associated with the plurality of medical devices, wherein the plurality of medical devices in the production line are individually moved adjacent to an antenna of the RFID reader by a conveyor; and for each identifier of the plurality of identifiers: determining, with at least one processor, whether that identifier is included in a list of expected identifiers; and in response to determining that that identifier is not included in the list of expected identifiers, controlling, with the at least one processor, the conveyor to remove a medical device of the plurality of medical devices associated with that identifier from the production line.
Clause 12. The method of clause 11, further comprising: for each medical device of the plurality of medical devices in the production line: detecting, with at least one sensor, when that medical device is in a predetermined location relative to the antenna of the RFID reader; in response to detecting, with the at least one sensor, that that medical device is in the predetermined location relative to the antenna of the RFID reader in the production line, transmitting, with the at least one sensor, a trigger signal to the RFID reader to trigger the RFID reader to attempt to read a RFID tag of the plurality of RFID tags on that medical device; and in response to receiving the trigger signal from the at least one sensor, attempting to read, with the RFID reader, the RFID tag on that medical device.
Clause 13. The method of clause 11 or 12, further comprising: in response to at least one attempt to read at least one RFID tag on at least one medical device that fails to read at least one identifier associated with the at least one medical device from the at least one RFID tag, controlling, with the at least one processor, the conveyor to remove the at least one medical device from the production line.
Clause 14. The method of any of clauses 11-13, wherein, for each identifier, determining, with the at least one processor, whether that identifier is included in the list of expected identifiers, includes: querying, with the at least one processor, based on that identifier, a database including the list of expected identifiers.
Clause 15. The method of any of clauses 11-14, wherein, for each identifier, the database including the list of expected identifiers is immediately queried, based on that identifier, in response to reading, with the RFID reader, that identifier.
Clause 16. The method of any of clauses 11-15, wherein the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the method further comprises: for each cycle of the series of cycles: after each medical device included in the subset of medical devices in that cycle is individually moved adjacent to the antenna of the RFID reader by the conveyor, querying, with the at least one processor, based on a subset of identifiers associated with that subset of medical devices in that cycle, the database including the list of expected identifiers.
Clause 17. The method of any of clauses 11-16, further comprising: obtaining, with the at least one processor, a batch number associated with the plurality of medical devices; and controlling, with the at least one processor, based on the batch number, the database to synchronize the list of expected identifiers with a further database that includes a plurality of lists of expected identifiers associated with a plurality of batch numbers.
Clause 18. The method of any of clauses 11-17, wherein the plurality of medical devices includes a plurality of syringes.
Clause 19. The method of any of clauses 11-18, wherein the plurality of identifiers includes a plurality of electronic product codes.
Clause 20. The method of any of clauses 11-19, further comprising: for each identifier of the plurality of identifiers: in response to determining that that identifier is included in the list of expected identifiers, controlling, with the at least one processor, the conveyor to maintain the medical device of the plurality of medical devices associated with that identifier in the production line.
Clause 21. A computer program product comprising at least one non-transitory computer-readable medium including program instructions that, when executed by at least one processor, cause the at least one processor to: receive, from a radio-frequency identification (RFID) reader, a plurality of identifiers read from a plurality of RFID tags on a plurality of medical devices in production line, wherein the plurality of medical devices in the production line are individually moved adjacent to an antenna of the RFID reader by a conveyor; and for each identifier of the plurality of identifiers: determine whether that identifier is included in a list of expected identifiers; and in response to determining that that identifier is not included in the list of expected identifiers, transmit, to the conveyor, a signal to remove a medical device of the plurality of medical devices associated with that identifier from the production line.
Clause 22. The computer program product of clause 21, wherein, for each medical device of the plurality of medical devices in the production line: at least one sensor detects when that medical device is in a predetermined location relative to the antenna of the RFID reader and transmits, in response to detecting that that medical device is in the predetermined location relative to the antenna of the RFID reader, a trigger signal to the RFID reader to trigger the RFID reader to attempt to read a RFID tag of the plurality of RFID tags on that medical device; and the RFID reader attempts to read the RFID tag on that medical device in response to receiving the trigger signal from the at least one sensor, and wherein the program instructions, when executed by the at least one processor, further cause the at least one processor to: for each medical device of the plurality of medical devices in the production line, receive, from the RFID reader, at least one of: (i) an indication of whether a read attempt associated with that medical device failed to read an identifier associated with that medical device, (ii) the identifier associated with that medical device, or any combination thereof.
Clause 23. The computer program product of clause 21 or 22, wherein the program instructions, when executed by the at least one processor, further cause the at least one processor to: in response to receiving an indication that the read attempt associated with that medical device failed, control the conveyor to remove that medical device from the production line.
Clause 24. The computer program product of any of clauses 21-23, wherein the program instructions, when executed by the at least one processor, further cause the at least one processor to determine, for each identifier, whether that identifier is included in the list of expected identifiers by: querying, based on that identifier, a database including the list of expected identifiers.
Clause 25. The computer program product of any of clauses 21-24, wherein the program instructions, when executed by the at least one processor, cause the at least one processor to immediately query, for each identifier, the database including the list of expected identifiers in response to receiving that identifier from the RFID reader.
Clause 26. The computer program product of any of clauses 21-25, wherein the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the program instructions, when executed by the at least one processor, further cause the at least one processor to: for each cycle of the series of cycles: after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.
Clause 27. The computer program product of any of clauses 21-26, wherein the program instructions, when executed by the at least one processor, further cause the at least one processor to: obtain a batch number associated with the plurality of medical devices; and control, based on the batch number, the database to synchronize the list of expected identifiers with a further database that includes a plurality of lists of expected identifiers associated with a plurality of batch numbers.
Clause 28. The computer program product of any of clauses 21-27, wherein the plurality of medical devices includes a plurality of syringes.
Clause 29. The computer program product of any of clauses 21-28, wherein the plurality of identifiers includes a plurality of electronic product codes.
Clause 30. The computer program product of any of clauses 21-29, wherein the program instructions, when executed by the at least one processor, further cause the at least one processor to: for each identifier of the plurality of identifiers: in response to determining that that identifier is included in the list of expected identifiers, control the conveyor to maintain the medical device of the plurality of medical devices associated with that identifier in the production line.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and the claims, the singular form of "a," "an," and "the" include plural referents unless the context clearly dictates otherwise.

### BRIEF DESCRIPTION OF THE DRAWINGS

Additional advantages and details are explained in greater detail below with reference to the exemplary embodiments that are illustrated in the accompanying schematic figures, in which:
FIG. 1 is a diagram of non-limiting embodiments or aspects of an environment in which systems, devices, products, apparatus, and/or methods, described herein, can be implemented;
FIG. 2 is a diagram of non-limiting embodiments or aspects of components of one or more devices and/or one or more systems of FIG. 1;
FIGS. 3A and 3B are a flowchart of non-limiting embodiments or aspects of a process for singulated medical devices on high-throughput machines;
FIG. 4 is a timing diagram of a cycle-based implementation of non-limiting embodiments or aspects of a process for singulated medical devices on high-throughput machines; and
FIG. 5 is a timing diagram of a continuous-based implementation of non-limiting embodiments or aspects of a process for singulated medical devices on high-throughput machines.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to embodiments or aspects as they are oriented in the drawing figures. However, it is to be understood that embodiments or aspects may assume various alternative variations and step sequences, except where expressly specified to the contrary. It is also to be understood that the specific devices and processes illustrated in the attached drawings, and described in the following specification, are simply non-limiting exemplary embodiments or aspects. Hence, specific dimensions and other physical characteristics related to the embodiments or aspects of the embodiments or aspects disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, within a range specified by a threshold, etc.

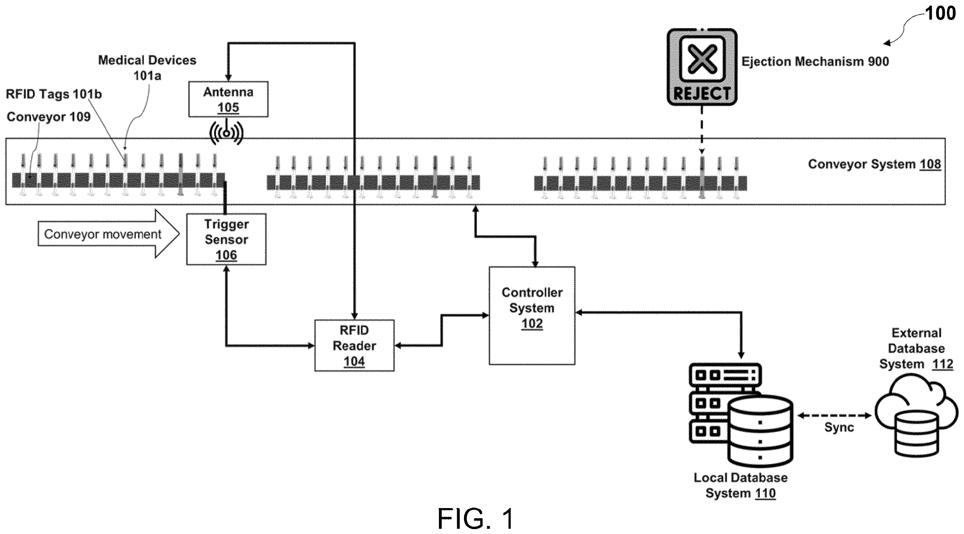

Referring now to FIG. 1, FIG. 1 is a diagram of an example environment 100 in which devices, systems, methods, and/or products described herein, may be implemented. As shown in FIG. 1, environment 100 may include a plurality of medical devices 101a including a plurality of RFID tags 101b, controller system 102, RFID reader 104 including antenna 105, trigger sensor 106, conveyor system 108 including conveyer 109 and/or ejection mechanism 900, local database system 110, and/or external database system 112.

The plurality of medical devices 101a may be arranged in a production line. For example, the plurality of medical devices 101a may include at least one of the following types of medical devices: syringes, autoinjectors, medication containers or vials, IV bags, or any combination thereof. As an example, the plurality of medical devices 101a may include a plurality of syringes.

The plurality of RFID tags 101b on the plurality medical devices 101a may include passive RFID tags, active RFID tags, or any combination thereof. The plurality of RFID tags 101b may store identifiers associated with the plurality of medical devices 101a. For example, each RFID tag 101b may store a unique identifier of the medical device 101a on which that RFID tag 101b is located. As an example, an identifier may include an electronic product code (EPC).

Controller system 102 may include one or more devices capable of receiving information and/or data from RFID reader 104, trigger sensor 106, conveyor system 108, local database system 110, and/or external database system 112 (e.g., via a communication network, via a direct wired and/or wireless connection, etc.) and/or communicating information and/or data to RFID reader 104, trigger sensor 106, conveyor system 108, local database system 110, and/or external database system 112 (e.g., via communication network, via a direct wired and/or wireless connection, etc.). For example, controller system 102 may include a computing device, such as a server, a group of servers, and/or other like devices. In some non-limiting embodiments or aspects, controller system 102 may include middleware, such as Message Queuing Telemetry Transport (MQTT) protocol-based middleware, and/or the like, configured to manage communication with and/or control of operations of RFID reader 104, trigger sensor 106, conveyor system 108 (e.g., a programmable logic controller (PLC) of conveyor system, 108 etc.), local database system 110, and/or external database system 112.

RFID reader 104 may include one or more devices capable of receiving information and/or data from controller system 102, trigger sensor 106, conveyor system 108, local database system 110, and/or external database system 112 (e.g., via a communication network, via a direct wired and/or wireless connection, etc.) and/or communicating information and/or data to controller system 102, trigger sensor 106, conveyor system 108, local database system 110, and/or external database system 112 (e.g., via communication network, via a direct wired and/or wireless connection, etc.).

RFID reader 104 may include a passive RFID reader, an active RFID reader, or any combination thereof. RFID reader 104 may be configured to read, from the plurality of RFID tags 101b on the plurality of medical devices 101a in the production line, identifiers associated with medical devices 101a. For example, RFID reader 104 may be configured to attempt to read an RFID tag 101b on a medical device 101a in response to receiving a trigger signal from trigger sensor 106.

Antenna 105 (e.g., a RFID near field antenna or coupling element, etc.) of RFID reader 104 may be located proximate to conveyor 109 such that, as conveyor 109 moves the plurality of medical devices 101a in the production line, the plurality of medical devices are moved one-by-one to be adjacent to (and/or to be paused adjacent to for a predetermined period of time) antenna 105 of RFID reader 104, and moved then past, antenna 105 of RFID reader 104. For example, a spacing between the plurality of medical devices 101a in the production line on conveyor 109 and/or a size of a magnetic or electromagnetic field generated by RFID reader 104 from antenna 105 may be configured such that only a single medical device of the plurality of medical devices 101a is located in the magnetic or electromagnetic field generated by RFID reader 104 from antenna 105 at any one time (e.g., such that only a single medical device of the plurality of medical devices 101a can be read by RFID reader 104 at any one time, etc.).

Trigger sensor 106 may include one or more devices capable of receiving information and/or data from controller system 102, RFID reader 104, conveyor system 108, local database system 110, and/or external database system 112 (e.g., via a communication network, via a direct wired and/or wireless connection, etc.) and/or communicating information and/or data to controller system 102, RFID reader 104, conveyor system 108, local database system 110, and/or external database system 112 (e.g., via communication network, via a direct wired and/or wireless connection, etc.).

Trigger sensor 106 may include at least one sensor (e.g., an optical sensor, a laser sensor, etc.) configured to detect or be trigged when an individual medical device is in a predetermined location relative to antenna 105 of the RFID reader 104 (e.g., when a medical device is within a field of detection or view of trigger sensor 106, etc.). Trigger sensor 106 may be located proximate or adjacent to antenna 105 of RFID reader 104 and/or conveyor 109 and/or at a position relative to antenna 105 of RFID reader 104 and/or conveyor 109 at which, when a medical device is in the predetermined location relative to antenna 105 of the RFID reader 104, the medical device is within the field of detection or view of trigger sensor 106 such that trigger sensor 106 is triggered or detects the medical device. In response to being triggered or detecting a medical device in the predetermined location relative to antenna 105 of the RFID reader 104, trigger sensor 106 may be configured to communicate or transmit a trigger signal to RFID reader 104 to trigger or cause RFID reader 104 to attempt to read a RFID tag on the medical device.

Conveyor system 108 may include one or more devices capable of receiving information and/or data from controller system 102, RFID reader 104, trigger sensor 106, local database system 110, and/or external database system 112 (e.g., via a communication network, via a direct wired and/or wireless connection, etc.) and/or communicating information and/or data to controller system 102, RFID reader 104, trigger sensor 106, local database system 110, and/or external database system 112 (e.g., via communication network, via a direct wired and/or wireless connection, etc.).

Conveyor system 108 may include conveyor 109 (e.g., a motorized belt conveyor, a motorized chute conveyor, a motorized roller conveyor, a motorized overhead conveyor, a motorized bucket conveyor, a motorized chain conveyor, a motorized trolley conveyor, etc.). Conveyor 109 may be configured to individually move (e.g., move one-by-one, etc.) medical devices 101a in the production line adjacent to and past antenna 105 of RFID reader 104. For example, medical devices 101a may be arranged on conveyor 109, and/or conveyor 109 may be configured to move medical devices 101a together in a same direction such that medical devices 101a are moved one-by-one adjacent to (and/or paused adjacent to for a predetermined period of time), and moved then past, antenna 105 of RFID reader 104. As an example, conveyor 109 may include one or more high throughput production machines and/or components thereof used in the pharmaceutical industry, such as a machine used for a fill and finish process, a machine used for an automated visual inspection process, a machine used for a labelling process, a machine used for a device assembly process, and/or the like.

Conveyor 109 may include an ejection mechanism 900 configured to remove (e.g., eject from, etc.) an individual medical device 101a from conveyor 109 and/or the production line. For example, ejection mechanism 900 may include an air blast generator, a pneumatic pusher, an overhead sweep, a pneumatic retractor, a pivot down/up and linear actuating ejector, and/or the like.

In some non-limiting embodiments or aspects, conveyor system 108 includes a programmable logic controller (PLC) configured to control one or more operations of conveyor 109. The PLC may be configured to track a sequential order of the plurality of medical devices 101a in the production line and/or on conveyor 109 via one or more shift registers and/or sensors according to existing manufacturing techniques.. The PLC may be configured to use the tracked sequential order to remove or eject individual ones of the plurality of medical devices 101a from the production line in response to ejection signals received from controller system 102 as described herein in more detail below.

Local database system 110 may include one or more devices capable of receiving information and/or data from controller system 102, RFID reader 104, trigger sensor 106, conveyor system 108, and/or external database system 112 (e.g., via a communication network, via a direct wired and/or wireless connection, etc.) and/or communicating information and/or data to controller system 102, RFID reader 104, trigger sensor 106, conveyor system 108, and/or external database system 112 (e.g., via communication network, via a direct wired and/or wireless connection, etc.). In some non-limiting embodiments or aspects, local database system 110 includes one or more local databases (e.g., local to and/or implemented by controller system 102, etc.). For example, local database system 110 may be configured to store a list of expected identifiers.

External database system 112 may include one or more devices capable of receiving information and/or data from controller system 102, RFID reader 104, trigger sensor 106, conveyor system 108, and/or local database system 110 (e.g., via a communication network, via a direct wired and/or wireless connection, etc.) and/or communicating information and/or data to controller system 102, RFID reader 104, trigger sensor 106, conveyor system 108, and/or local database system 110 (e.g., via communication network, via a direct wired and/or wireless connection, etc.). In some non-limiting embodiments or aspects, external database system 112 includes one or more external databases (e.g., external to and/or not implemented by controller system 102, etc.). For example, external database system 112 may be configured to store a plurality of lists of expected identifiers associated with a plurality of batch numbers.

The number and arrangement of systems and devices shown in FIG. 1 is provided as an example. There can be additional systems and/or devices, fewer systems and/or devices, different systems and/or devices, or differently arranged systems and/or devices than those shown in FIG. 1. Furthermore, two or more systems or devices shown in FIG. 1 can be implemented within a single system or a single device, or a single system or a single device shown in FIG. 1 can be implemented as multiple, distributed systems or devices. Additionally, or alternatively, a set of systems or a set of devices (e.g., one or more systems, one or more devices, etc.) of environment 100 can perform one or more functions described as being performed by another set of systems or another set of devices of environment 100.

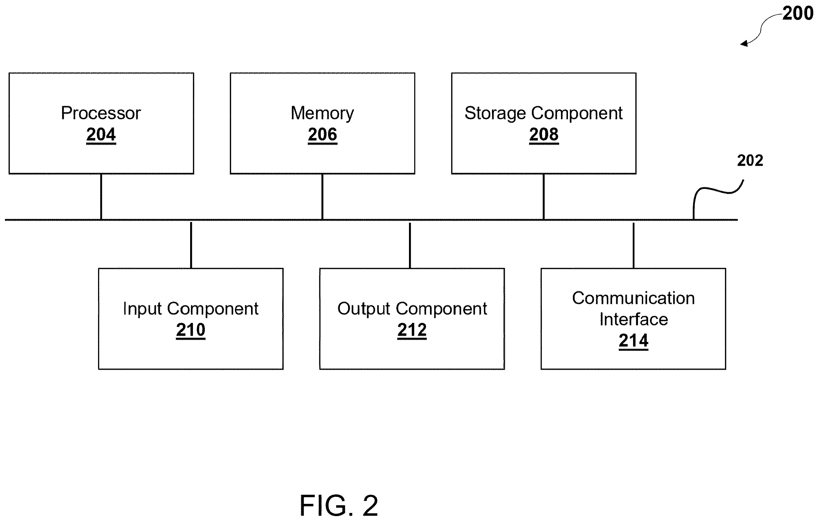

Referring now to FIG. 2, FIG. 2 is a diagram of example components of a device 200. Device 200 may correspond to one or more devices of controller system 102, RFID reader 104 (e.g., one or more devices of a system of RFID reader 104, etc.), trigger sensor 106 (e.g., one or more devices of a system of trigger sensor 106, etc.), one or more devices of conveyor system 108, one or more devices of local database system 110, and/or one or more devices of external database system 112. In some non-limiting embodiments or aspects, one or more devices of controller system 102, RFID reader 104 (e.g., one or more devices of a system of RFID reader 104, etc.), trigger sensor 106 (e.g., one or more devices of a system of trigger sensor 106, etc.), one or more devices of conveyor system 108, one or more devices of local database system 110, and/or one or more devices of external database system 112 may include at least one device 200 and/or at least one component of device 200. As shown in FIG. 2, device 200 may include bus 202, processor 204, memory 206, storage component 208, input component 210, output component 212, and communication interface 214.

Bus 202 may include a component that permits communication among the components of device 200. In some non-limiting embodiments or aspects, processor 204 may be specifically configured to perform one or more of the mixing aspects described in hardware, software, or a combination of hardware and software. For example, processor 204 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 206 may include random access memory (RAM), read-only memory (ROM), and/or another type of dynamic or static storage device (e.g., flash memory, magnetic memory, optical memory, etc.) that stores information and/or instructions for use by processor 204.

Storage component 208 may store information and/or software related to the operation and use of device 200. For example, storage component 208 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 210 may include a component that permits device 200 to receive information, such as via user input (e.g., a touch screen display, a keyboard, a keypad, a mouse, a button, a switch, a microphone, etc.). Additionally or alternatively, input component 210 may include a sensor for sensing information (e.g., a global positioning system (GPS) component, an accelerometer, a gyroscope, an actuator, etc.). Output component 212 may include a component that provides output information from device 200 (e.g., a display, a speaker, one or more light-emitting diodes (LEDs), etc.).

Communication interface 214 may include a transceiver-like component (e.g., a transceiver, a separate receiver and transmitter, etc.) that enables device 200 to communicate with other devices, such as via a wired connection, a wireless connection, or a combination of wired and wireless connections. Communication interface 214 may permit device 200 to receive information from another device and/or provide information to another device. For example, communication interface 214 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 200 may perform one or more processes described herein. Device 200 may perform these processes based on processor 204 (e.g., a central processing unit (CPU), a graphics processing unit (GPU), etc.) executing software instructions stored by a computer-readable medium, such as memory 206 and/or storage component 208. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 206 and/or storage component 208 from another computer-readable medium or from another device via communication interface 214. When executed, software instructions stored in memory 206 and/or storage component 208 may cause processor 204 to perform one or more processes described herein. Additionally or alternatively, hardwired circuitry may be used in place of or in combination with software instructions to perform one or more processes described herein.

Memory 206 and/or storage component 208 may include data storage or one or more data structures (e.g., a database, etc.). Device 200 may be capable of receiving information from, storing information in, communicating information to, or searching information stored in the data storage or one or more data structures in memory 206 and/or storage component 208.

The number and arrangement of components shown in FIG. 2 are provided as an example. In some non-limiting embodiments or aspects, device 200 may include additional components, fewer components, different components, or differently arranged components than those shown in FIG. 2. Additionally or alternatively, a set of components (e.g., one or more components) of device 200 may perform one or more functions described as being performed by another set of components of device 200.

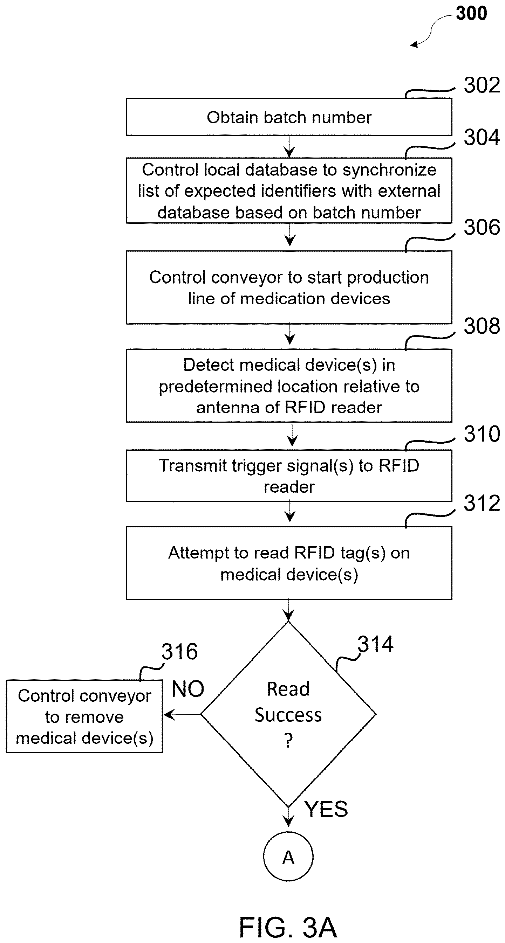

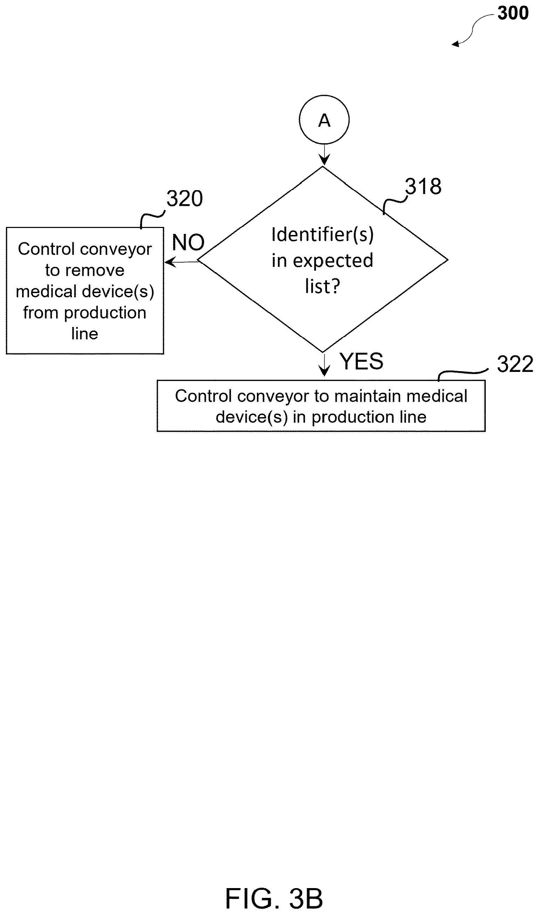

Referring now to FIGS. 3A and 3B, FIGS. 3A and 3B are a flowchart of non-limiting embodiments or aspects of a process 300 for singulated medical devices on high-throughput machines. In some non-limiting embodiments or aspects, one or more of the steps of process 300 may be performed (e.g., completely, partially, etc.) by controller system 102 (e.g., one or more devices of controller system 102, etc.). In some non-limiting embodiments or aspects, one or more of the steps of process 300 may be performed (e.g., completely, partially, etc.) by another device or a group of devices separate from or including controller system 102, such as RFID reader 104 (e.g., one or more devices of a system of RFID reader 104, etc.), trigger sensor 106 (e.g., one or more devices of a system of trigger sensor 106, etc.), conveyor system 108 (e.g., one or more devices of conveyer system 108, etc.), local database system 110 (e.g., one or more devices of local database system 110, etc.), and/or external database system 112 (e.g., one or more devices of external database system 110, etc.).

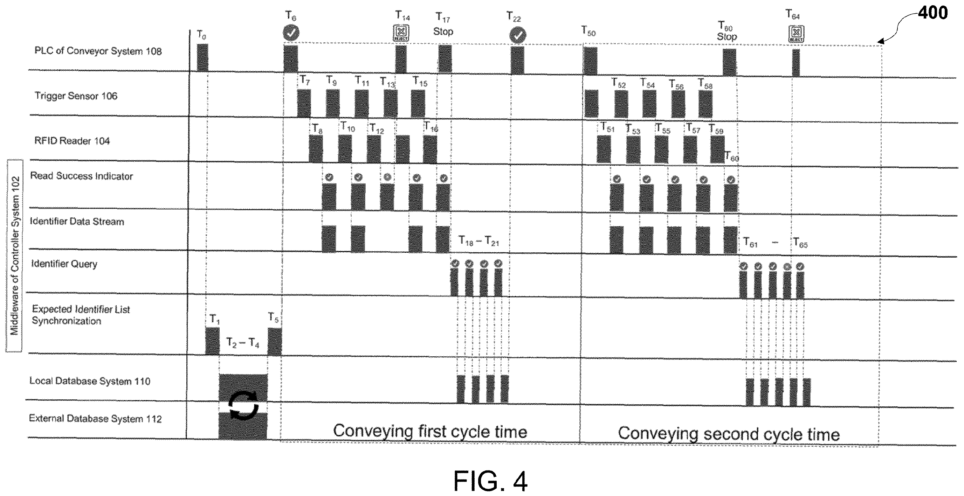

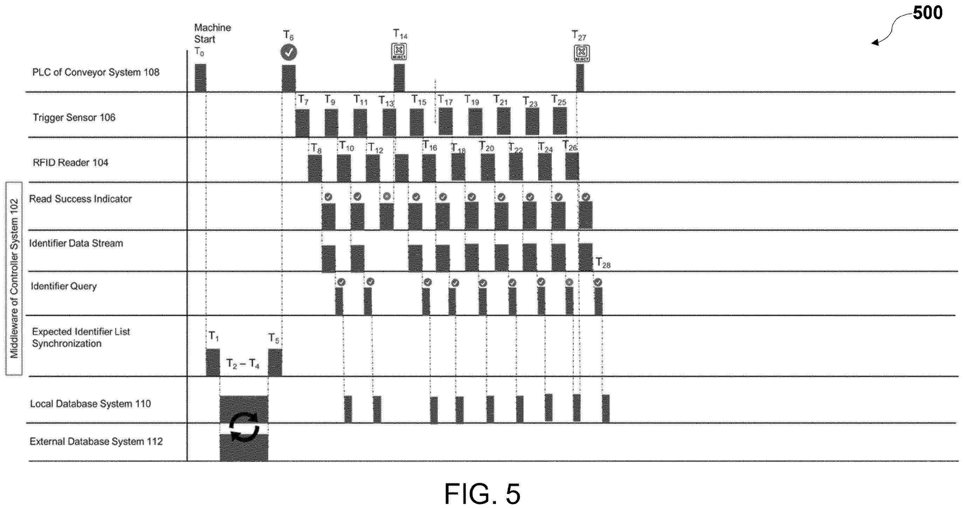

As shown in FIG. 3A, at step 302, process 300 includes obtaining a batch number. For example, controller system 102 may obtain a batch number associated with a plurality of medical devices 101a (e.g., a plurality of syringes, etc.) in a production line. As an example, and referring also to FIGS. 4 and 5, which are timing diagrams of a cycle-based implementation 400 (e.g., stop-and-go conveying, etc.) and a continuous-based implementation 500 (e.g., continuous conveying, etc.), respectively, of non-limiting embodiments or aspects of a process for singulated medical devices on high-throughput machines, at a reference time T₀, conveyor system 108 (e.g., a PLC of conveyor system 108, etc.) may transmit a signal to controller system 102 (e.g., to middleware of controller system 102, etc.) that requests a batch number of the plurality of medical devices 101a in the production line. In such an example, controller system 102 may provide a prompt via a user interface (e.g., via output component 212, etc.) to a user to enter the batch number, and/or conveyor system 108 may receive the batch number as user input via the user interface.

As shown in FIG. 3A, at step 304, process 300 includes controlling a local database to synchronize a list of expected identifiers with an external database based on a batch number. For example, controller system 102 may control, based on the batch number, local database system 110 to synchronize a list of expected identifiers (e.g., expected EPCs, etc.) with external database system 112. As an example, external database system 112 may store a plurality of lists of expected identifiers associated with a plurality of batch numbers associated with a plurality of batches of medical devices. In such an example, and referring again to FIGS. 4 and 5, at reference time T₁, controller system 102 (e.g., middleware of controller system 102, etc.) may send a query signal local database system 110 to cause local database system 110 to synchronize with external database system 112 (e.g., download from, update from, store from, etc.) a list of expected identifiers associated with the batch number of the plurality of medical devices 101a in the production line. In response to receiving the query signal, at reference times T₂ - T₄, local database system 110 may synchronize with external database system 112 the list of expected identifiers associated with the batch number of the plurality of medical devices 101a in the production line. At reference time T₅, controller system 102 (e.g., middleware of controller system 102, etc.) may receive a signal from local database system 110 that confirms the synchronization.

As shown in FIG. 3A, at step 306, process 300 includes controlling a conveyor to start a production line of medical devices. For example, controller system 102 may control conveyor system 108 to start the production line including the plurality of medical devices 101a, for example, to cause conveyor 109 to start conveyor movement to move the plurality of medical devices 101a in the production line adjacent to and past antenna 105 of RFID reader 104. As an example, and referring again to FIGS. 4 and 5, at reference time T6, controller system 102 (e.g., middleware of controller system 102, etc.) may send a signal to conveyor system 108 (e.g., a PLC of conveyor system 108, etc.) to confirm the synchronization and cause conveyor system 108 to control conveyor 109 to start conveyor movement. In such an example, controller system 102 (e.g., middleware of controller system 102, etc.) may send a signal to RFID reader to arm RFID reader 104.

As shown in FIG. 3A, at step 308, process 300 includes detecting a medical device in a predetermined location relative to an antenna of a RFID reader. For example, for each medical device of the plurality of medical devices 101a in the production line, trigger sensor 106 may detect when that medical device is in a predetermined location relative to antenna 105 of RFID reader 104.

As shown in FIG. 3A, at step 310, process 300 includes transmitting a trigger signal to a RFID reader. For example, for each medical device of the plurality of medical devices 101a in the production line, trigger sensor 106 may, in response to detecting that the medical device is in the predetermined location relative to antenna 105 of RFID reader 104 in the production line, transmit a trigger signal to RFID reader to trigger or cause RFID reader 104 to attempt to read a RFID tag of the plurality of RFID tags 101b on that medical device. As an example, and referring specifically to FIG. 4, at reference times T₇, T₉, T₁₁, T₁₃, and T₁₅ in a first cycle in a series of cycles of cycle-based implementation 400 of non-limiting embodiments or aspects, trigger sensor 106 may be respectively triggered by and/or respectively transmit trigger signals to RFID reader 104 corresponding to a first subset of medical devices of the plurality of medical devices 101a in the production line being individually moved adjacent to antenna 105 of the RFID reader 104 by conveyor 109, and/or at reference times T₅₀, T₅₂, T₅₄, T₅₆, and T₅₈ in a second cycle in the series of cycles of cycle-based implementation 400 of non-limiting embodiments or aspects, trigger sensor 106 may be respectively triggered by and/or transmit trigger signals to RFID reader 104 corresponding to a second or next subset of medical devices of the plurality of medical devices 101a in the production line being individually moved adjacent to antenna 105 of the RFID reader 104 by conveyor 109. As an example, and referring specifically to FIG. 5, at reference times T₇, T₉, T₁₁, T₁₃, T₁₅, T₁₇, T₁₉, T₂₁, T₂₃, and T₂₅ of continuous-based implementation 500 of non-limiting embodiments or aspects, trigger sensor 106 may be respectively triggered by and/or transmit trigger signals to RFID reader 104 corresponding to the plurality of medical devices 101a in the production line being individually moved adjacent to antenna 105 of the RFID reader 104 by conveyor 109.

As shown in FIG. 3A, at step 312, process 300 includes attempting to read an RFID tag on a medical device. For example, for each medical device of the plurality of medical devices 101a in the production line, RFID reader 104 may, in response to receiving the trigger signal from trigger sensor 106 (e.g., corresponding to that medical device, etc.), attempt to read the RFID tag on that medical device. As an example, RFID reader 104 may transmit radio waves via antenna 105 to attempt to activate the RFID tag, which, if operating correctly, in response may transmit a signal, which may include an identifier associated with that medical device, back to antenna 105 to be read by RFID reader 104. As an example, RFID reader 104 may read, from the plurality of RFID tags 101b on the plurality of medical devices 101a in the production line, a plurality of identifiers (e.g., a plurality of EPCs, etc.) associated with the plurality of medical devices 101a (e.g. as the plurality of medical devices 101a in the production line are individually moved adjacent to antenna 105 of RFID reader 104 by conveyor 109, etc.). In such an example, for each medical device of the plurality of medical devices 101a in the production line, RFID reader 104 may transmit or stream, to controller system 102 (e.g., to middleware of controller system 102, etc.) an indication of whether the read attempt of the RFID tag on that medical device was successful in reading information or data from the RFID tag (e.g. an indication of whether that attempt was successful in reading an identifier associated with the medical device, etc.) and/or the information or data read from the RFID tag in that attempt (e.g., the identifier, etc.).

Referring again specifically to FIG. 4, in the first cycle in the series of cycles of cycle-based implementation 400 of non-limiting embodiments or aspects, at reference times T₈, T₁₀, T₁₂, T₁₄, and T₁₆, RFID reader 104 may, in response to receiving the trigger signals transmitted by trigger sensor 106 at reference times T₇, T₉, T₁₁, T₁₃, and T₁₅, respectively attempt to read RFID tags of a first subset of RFID tags on the first subset of medical devices of the plurality of medical devices 101a in the production line, and/or in the second or next cycle in the series of cycles of cycle-based implementation 400 of non-limiting embodiments or aspects, at reference times T₅₁, T₅₃, T₅₅, T₅₇, and T₅₉, RFID reader 104 may, in response to receiving the trigger signals transmitted by trigger sensor 106 at reference times T₅₀, T₅₂, T₅₆, and T₅₈, respectively attempt to read RFID tags of a second or next subset of RFID tags on the second or next subset of medical devices of the plurality of medical devices 101a in the production line. In such an example, at reference times T₉, T₁₁, T₁₃, T₁₅, and T₁₇ in the first cycle, RFID reader 104 may respectively transmit or stream, to controller system 102 (e.g., to middleware of controller system 102, etc.) indications of whether the read attempts at reference times T₈, T₁₀, T₁₂, T₁₄, and T₁₆ were successful in reading identifiers from the first subset of RFID tags on the first subset of medical devices of the plurality of medical devices 101a and/or the identifiers themselves, and/or at reference times T₅₂, T₅₆, T₅₈, and T₆₀ in the second cycle, RFID reader 104 may respectively transmit or stream, to controller system 102 (e.g., to middleware of controller system 102, etc.) indications of whether the read attempts at reference times T₅₁, T₅₃, T₅₅, T₅₇, and T₅₉ were successful in reading identifiers from the second or next subset of RFID tags on the second or next subset of medical devices of the plurality of medical devices 101a and/or the identifiers themselves.

Referring again specifically to FIG. 5, at reference times T₈, T₁₀, T₁₂, T₁₄, T₁₆, T₁₈, T₂₀, T₂₂, T₂₄, and T₂₆ of continuous-based implementation 500 of non-limiting embodiments or aspects, RFID reader 104 may, in response to receiving the trigger signals transmitted by trigger sensor 106 at reference times T₇, T₉, T₁₁, T₁₃, T₁₅, T₁₇, T₁₉, T₂₁, T₂₃, and T₂₅, respectively attempt to read RFID tags of the plurality of RFID tags 101b on the plurality of medical devices 101a in the production line. In such an example, at reference times T₉, T₁₁, T₁₃, T₁₅, T₁₇, T₁₉, T₂₁, T₂₃, T₂₅, and T₂₇, RFID reader 104 may respectively transmit or stream, to controller system 102 (e.g., to middleware of controller system 102, etc.) indications of whether the read attempts at reference times T₈, T₁₀, T₁₂, T₁₄, T₁₆, T₁₈, T₂₀, T₂₂, T₂₄, and T₂₆ were successful in reading identifiers from the plurality of medical devices 101a and/or the identifiers themselves.

As shown in FIG. 3A, at step 314, process 300 includes determining whether an attempt to read an RFID tag on a medical device successfully read an identifier from the RFID tag. For example, for each medical device of the plurality of medical devices 101a in the production line, controller 102 may determine, based on the indication of whether the read attempt of the RFID tag on that medical device was successful in reading information or data from the RFID tag received from RFID reader 104, whether the attempt to read the RFID tag on that medical device successfully read aa identifier from the RFID tag.

As shown in FIG. 3A, at step 316, process 300 includes, in response to determining that an attempt to read an RFID tag on a medical device failed to read an identifier from the RFID tag, controlling a conveyor to remove the medical device from the production line. For example, in response to at least one attempt to read at least one RFID tag on at least one medical device that fails to read at least one identifier associated with the at least one medical device from the at least one RFID tag, controller system 102 (e.g., middleware of controller system 102, etc.) may control (e.g., via conveyor system 108, via a PLC of conveyor system 108, etc.) conveyor 109 to remove the at least one medical device from the production line. As an example, for each medical device of the plurality of medical devices 101a in the production line, in response to receiving an indication that the read attempt of the RFID tag on that medical device was not successful (and/or in response to not receiving the identifier itself within a predetermined time window, etc.), controller system 102 (e.g., middleware of controller system 102, etc.) may transmit a signal to conveyor system 108 (e.g., to a PLC of conveyor system 108, etc.) that causes conveyor system 108 to control conveyor 109 to remove that medical device from the production line (e.g., a PLC of conveyor system 108 conveyor system 108 may control ejection mechanism 900 to remove (e.g., eject from, etc.) that medical device from the production line.

Referring again specifically to FIG. 4, in the first cycle in the series of cycles of cycle-based implementation 400 of non-limiting embodiments or aspects, in response to receiving, from RFID reader 104 at reference time T₁₃, an indication that that the read attempt at reference time T₁₂ of the RFID tag on the corresponding medical device was not successful , controller system 102 (e.g., middleware of controller system 102, etc.) may transmit, at reference time T14, a signal to conveyor system 108 (e.g., to a PLC of conveyor system 108, etc.) that causes conveyor system 108 to control ejection mechanism 900 of conveyor 109 to remove that medical device from the production line.

Referring again specifically to FIG. 5, in continuous-based implementation 500 of non-limiting embodiments or aspects, in response to receiving, from RFID reader 104 at reference time T₁₃, an indication that that the read attempt at reference time T₁₂ of the RFID tag on the corresponding medical device was not successful, controller system 102 (e.g., middleware of controller system 102, etc.) may transmit, at reference time T14, a signal to conveyor system 108 (e.g., to a PLC of conveyor system 108, etc.) that causes conveyor system 108 to control ejection mechanism 900 of conveyor 109 to remove that medical device from the production line.

As shown in FIG. 3B, at step 318, process 300 includes, in response to determining that an attempt to read an RFID tag on a medical device successfully read an identifier from the RFID tag, determining whether that identifier is included in a list of expected identifiers. For example, for each identifier of the plurality of identifiers, controller system 102 may determine whether that identifier is included in a list of expected identifiers. As an example, for each identifier of the plurality of identifiers, controller system 102 (e.g., middleware of controller system 102, etc.) may determine whether that identifier is included in the list of expected identifiers by querying, based on that identifier, local database system 110 that includes the list of expected identifiers. In such an example, local database system 110 may return a response to the query that indicates whether that identifier is included in the list of expected identifiers.

In some non-limiting embodiments or aspects, the plurality of medical devices 101a in the production line may be individually moved (e.g., moved one-by-one, etc.) adjacent to antenna 105 of RFID reader 104 by conveyor 109 in a series of cycles, and/or each cycle of the series of cycles may include a subset of medical devices of the plurality of medical devices 101a in the production line being individually moved adjacent to antenna 105 of RFID reader 104 by conveyor 109. For each cycle of the series of cycles, after each medical device included in the subset of medical devices in that cycle is individually moved adjacent to antenna 105 of RFID reader 104 by conveyor 109 (and/or before initiating a next cycle after that cycle, etc.), controller system 102 (e.g., middleware of controller system 102, etc.) may query, based on a subset of identifiers associated with that subset of medical devices in that cycle, local database system 110 including the list of expected identifiers. As an example, and referring again specifically to FIG. 4, in the first cycle in the series of cycles of cycle-based implementation 400 of non-limiting embodiments or aspects, controller system 102 (e.g., middleware of controller system 102, etc.) may query, at reference times T₁₈ - T₂₁, based on the subset of identifiers associated with that subset of medical devices in that cycle, local database system 110 including the list of expected identifiers after receiving, at reference times T₉, T₁₁, T₁₃, T₁₅, and T₁₇ in the first cycle, the indications of whether the read attempts at reference times T₈, T₁₀, T₁₂, T₁₄, and T₁₆ were successful in reading identifiers from the first subset of RFID tags on the first subset of medical devices of the plurality of medical devices 101a and/or the identifiers themselves, for example, after transmitting a stop signal from controller system 102 (e.g., from middleware of controller system 102, etc.) to conveyor system 108 (e.g., to a PLC of conveyor system 108, etc.) at reference time T₁₇ to control conveyor 109 to stop movement of the plurality of medical devices 101a in the production line. In such an example, controller system 102 (e.g., middleware of controller system 102, etc.) may not query (e.g., at reference times T₁₈ - T₂₁, etc.), based on the attempted RFID read at reference time T₁₂, local database system 110 including the list of expected identifiers because controller system 102 (e.g., middleware of controller system 102, etc.) received, from RFID reader 104 at reference time T₁₃, the indication that that the read attempt at reference time T₁₂ of the RFID tag on the corresponding medical device was not successful (e.g., because controller system 102 did not receive an identifier corresponding to that medical device, etc.).

As an example, and still referring specifically to FIG. 4, in the second or next cycle in the series of cycles of cycle-based implementation 400 of non-limiting embodiments or aspects, controller system 102 (e.g., middleware of controller system 102, etc.) may query, at reference times T₆₂ - T₆₅ based on the subset of identifiers associated with that subset of medical devices in that cycle, local database system 110 including the list of expected identifiers after receiving, at reference times T₅₂, T₅₆, T₅₈, and T₆₀ in the second cycle, the indications of whether the read attempts at reference times T₅₁, T₅₃, T₅₅, T₅₇, and T₅₉ were successful in reading identifiers from the second or next subset of RFID tags on the second or next subset of medical devices of the plurality of medical devices 101a and/or the identifiers themselves, for example, after transmitting a stop signal from controller system 102 (e.g., from middleware of controller system 102, etc.) to conveyor system 108 (e.g., to a PLC of conveyor system 108, etc.) at reference time T₆₀ to control conveyor 109 to stop movement of the plurality of medical devices 101a in the production line.

In some non-limiting embodiments or aspects, for each identifier of the plurality of identifiers read by RFID reader 104, controller system 102 (e.g., middleware of controller system 102, etc.) may immediately query, based on that identifier, local database system 110 including the list of expected identifiers, in response to reading, with RFID reader 104, that identifier (e.g., in response controller system 102 receiving that identifier from RFID reader 104, etc.). For example, and referring again specifically to FIG. 5, in continuous-based implementation 500 of non-limiting embodiments or aspects, controller system 102 (e.g., middleware of controller system 102, etc.) may query, at reference time T₁₀, based on the identifier received at reference time T₉, local database system 110 including the list of expected identifiers immediately in response to reading, with RFID reader 104, that identifier (e.g., immediately in response controller system 102 receiving that identifier from RFID reader 104 at reference time T₉, etc.). As an example, controller system 102 (e.g., middleware of controller system 102, etc.) may query, at reference times T₁₀, T₁₂, T₁₆, T₁₈, T₂₀, T₂₂, T₂₄, T₂₆, and T₂₈ based on the identifiers respectively received at reference times T₇, T₉, T₁₃, T₁₅, T₁₇, T₁₉, T₂₁, T₂₃, T₂₅, and T₂₇, local database system 110 including the list of expected identifiers immediately in response to reading, with RFID reader 104, each identifier (e.g., immediately in response to controller system 102 receiving each identifier from RFID reader 104 at reference times T₇, T₉, T₁₃, T₁₅, T₁₇, T₁₉, T₂₁, T₂₃, T₂₅, and T₂₇, etc.). For example, controller system 102 (e.g., middleware of controller system 102, etc.) may receive, at reference times T₁₀, T₁₂, T₁₆, T₁₈, T₂₀, T₂₂, T₂₄, T₂₆, and T₂₈. In such an example, controller system 102 (e.g., middleware of controller system 102, etc.) may not query (e.g., at reference time T₁₄, etc.), based on the attempted RFID read at reference time T₁₂, local database system 110 including the list of expected identifiers because controller system 102 (e.g., middleware of controller system 102, etc.) received, from RFID reader 104 at reference time T₁₃, the indication that that the read attempt at reference time T₁₂ of the RFID tag on the corresponding medical device was not successful (e.g., because controller system 102 did not receive an identifier corresponding to that medical device, etc.).

As shown in FIG. 3B, at step 320, process 300 includes, in response to determining that an identifier is not included in a list of expected identifiers, controlling a conveyor to remove a medical device of the plurality of medical devices associated with that identifier from the production line. For example, for each identifier of the plurality of identifiers, controller system 102 (e.g., middleware of controller system 102, etc.) may, in response to determining that that identifier is not included in the list of expected identifiers, control conveyor 109 to remove a medical device of the plurality of medical devices 101a associated with that identifier from the production line. As an example, for each identifier of the plurality of identifiers, controller system 102 (e.g., middleware of controller system 102, etc.) may, in response to determining that that identifier is not included in the list of expected identifiers, transmit a signal to conveyor system 108 (e.g., to a PLC of conveyor system 108, etc.) that causes conveyor system 108 to control conveyor 109 to remove that medical device from the production line (e.g., the PLC of conveyor system 108 may control ejection mechanism 900 to remove (e.g., eject, etc.) that medical device from the production line.

For example, and referring again specifically to FIG. 4, in the second or next cycle in the series of cycles of cycle-based implementation 400 of non-limiting embodiments or aspects, controller system 102 (e.g., middleware of controller system 102, etc.) may, in response to determining at reference times T₆₁ - T₆₅ that the identifier received at reference time T₅₈ is not included in the list of expected identifiers, transmit, at reference time T₆₄, a signal to conveyor system 108 (e.g., to a PLC of conveyor system 108, etc.) that causes conveyor system 108 to control conveyor 109 to remove the corresponding medical device from the production line (e.g., the PLC of conveyor system 108 may control ejection mechanism 900 to remove (e.g., eject, etc.) the corresponding medical device from the production line.

For example, and referring again specifically to FIG. 5, in continuous-based implementation 500 of non-limiting embodiments or aspects, controller system 102 (e.g., middleware of controller system 102, etc.) may, in response to determining at reference time T₂₆ that the identifier received at reference time T₂₅ is not included in the list of expected identifiers, transmit, at reference time T₂₇, a signal to conveyor system 108 (e.g., to a PLC of conveyor system 108, etc.) that causes conveyor system 108 to control conveyor 109 to remove the corresponding medical device from the production line (e.g., the PLC of conveyor system 108 may control ejection mechanism 900 to remove (e.g., eject, etc.) the corresponding medical device from the production line.

As shown in FIG. 3B, at step 322, process 300 includes, in response to determining that an identifier is included in a list of expected identifiers, controlling a conveyor to maintain a medical device of the plurality of medical devices associated with that identifier from the production line. For example, for each identifier of the plurality of identifiers, controller system 102 (e.g., middleware of controller system 102, etc.) may, in response to determining that that identifier is included in the list of expected identifiers, control conveyor 109 to maintain the medical device of the plurality of medical devices 101a associated with that identifier in the production line. As an example, for each identifier of the plurality of identifiers, controller system 102 (e.g., middleware of controller system 102, etc.) may not, in response to determining that that identifier is included in the list of expected identifiers, transmit a signal to conveyor system 108 (e.g., to a PLC of conveyor system 108, etc.) that causes conveyor system 108 to control conveyor 109 to remove that medical device from the production line.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within the spirit and scope of the appended claims. For example, it is to be understood that the present disclosure contemplates that, to the extent possible, one or more features of any embodiment or aspect can be combined with one or more features of any other embodiment or aspect. In fact, many of these features can be combined in ways not specifically recited in the claims and/or disclosed in the specification. Although each dependent claim listed below may directly depend on only one claim, the disclosure of possible implementations includes each dependent claim in combination with every other claim in the claim set.

## Claims

1. A system, comprising:
a radio-frequency identification (RFID) reader configured to read, from a plurality of RFID tags on a plurality of medical devices in a production line, a plurality of identifiers associated with the plurality of medical devices;
a conveyor configured to individually move the plurality of medical devices in the production line adjacent to an antenna of the RFID reader; and
at least one processor coupled to a memory and configured to:
for each identifier of the plurality of identifiers:
determine whether that identifier is included in a list of expected identifiers; and
in response to determining that that identifier is not included in the list of expected identifiers, control the conveyor to remove a medical device of the plurality of medical devices associated with that identifier from the production line.

2. The system of claim 1, further comprising:
at least one sensor configured to detect when an individual medical device is in a predetermined location relative to the antenna of the RFID reader,
wherein, for each medical device of the plurality of medical devices in the production line:
the at least one sensor is configured to transmit a trigger signal to the RFID reader to cause the RFID reader to attempt to read a RFID tag of the plurality of RFID tags on that medical device in response to detecting that that medical device is in the predetermined location relative to the antenna of the RFID reader in the production line; and
the RFID reader is configured to attempt to read the RFID tag on that medical device in response to receiving the trigger signal from the at least one sensor.

3. The system of claim 2, wherein, in response to at least one attempt to read at least one RFID tag on at least one medical device that fails to read at least one identifier associated with the at least one medical device from the at least one RFID tag, the at least one processor is further configured to control the conveyor to remove the at least one medical device from the production line.

4. The system of claim 1, further comprising:
a local database configured to store the list of expected identifiers,
wherein, for each identifier, the at least one processor is configured to determine whether that identifier is included in the list of expected identifiers by querying, based on that identifier, the local database including the list of expected identifiers.

5. The system of claim 4, wherein, for each identifier, the at least one processor is configured to immediately query, based on that identifier, the local database including the list of expected identifiers in response to reading, with the RFID reader, that identifier.

6. The system of claim 4, wherein the conveyor is configured to individually move the plurality of medical devices in the production line adjacent to the antenna of the RFID reader in a series of cycles, wherein each cycle of the series of cycles includes a subset of medical devices of the plurality of medical devices in the production line being individually moved adjacent to the antenna of the RFID reader by the conveyor, and wherein the at least one processor is further configured to:
for each cycle of the series of cycles:
after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.

7. The system of claim 4, wherein the at least one processor is further configured to:
obtain a batch number associated with the plurality of medical devices; and
control, based on the batch number, the local database to synchronize the list of expected identifiers with an external database that includes a plurality of lists of expected identifiers associated with a plurality of batch numbers.

8. The system of claim 1, wherein the plurality of medical devices includes a plurality of syringes.

9. The system of claim 1, wherein the plurality of identifiers includes a plurality of electronic product codes.

10. The system of claim 1, wherein the at least one processor is further configured to:
for each identifier of the plurality of identifiers:
in response to determining that that identifier is included in the list of expected identifiers, control the conveyor to maintain the medical device of the plurality of medical devices associated with that identifier in the production line.

11. A method, comprising:
reading, with a radio-frequency identification (RFID) reader, from a plurality of RFID tags on a plurality of medical devices in a production line, a plurality of identifiers associated with the plurality of medical devices, wherein the plurality of medical devices in the production line are individually moved adjacent to an antenna of the RFID reader by a conveyor; and
for each identifier of the plurality of identifiers:
determining, with at least one processor, whether that identifier is included in a list of expected identifiers; and
in response to determining that that identifier is not included in the list of expected identifiers, controlling, with the at least one processor, the conveyor to remove a medical device of the plurality of medical devices associated with that identifier from the production line.

12. The method of claim 11, further comprising:
for each medical device of the plurality of medical devices in the production line:
detecting, with at least one sensor, when that medical device is in a predetermined location relative to the antenna of the RFID reader;
in response to detecting, with the at least one sensor, that that medical device is in the predetermined location relative to the antenna of the RFID reader in the production line, transmitting, with the at least one sensor, a trigger signal to the RFID reader to trigger the RFID reader to attempt to read a RFID tag of the plurality of RFID tags on that medical device; and
in response to receiving the trigger signal from the at least one sensor, attempting to read, with the RFID reader, the RFID tag on that medical device.

13. The method of claim 12, further comprising:
in response to at least one attempt to read at least one RFID tag on at least one medical device that fails to read at least one identifier associated with the at least one medical device from the at least one RFID tag, controlling, with the at least one processor, the conveyor to remove the at least one medical device from the production line.

14. The method of claim 11, wherein, for each identifier, determining, with the at least one processor, whether that identifier is included in the list of expected identifiers, includes:
querying, with the at least one processor, based on that identifier, a database including the list of expected identifiers.

15. The method of claim 14, wherein, for each identifier, the database including the list of expected identifiers is immediately queried, based on that identifier, in response to reading, with the RFID reader, that identifier.

16. The method of claim 14, wherein the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the method further comprises:
for each cycle of the series of cycles:
after each medical device included in the subset of medical devices in that cycle is individually moved adjacent to the antenna of the RFID reader by the conveyor, querying, with the at least one processor, based on a subset of identifiers associated with that subset of medical devices in that cycle, the database including the list of expected identifiers.

17. The method of claim 14, further comprising:
obtaining, with the at least one processor, a batch number associated with the plurality of medical devices; and
controlling, with the at least one processor, based on the batch number, the database to synchronize the list of expected identifiers with a further database that includes a plurality of lists of expected identifiers associated with a plurality of batch numbers.

18. The method of claim 11, wherein the plurality of medical devices includes a plurality of syringes.

19. The method of claim 11, wherein the plurality of identifiers includes a plurality of electronic product codes.

20. The method of claim 11, further comprising:
for each identifier of the plurality of identifiers:
in response to determining that that identifier is included in the list of expected identifiers, controlling, with the at least one processor, the conveyor to maintain the medical device of the plurality of medical devices associated with that identifier in the production line.

21. A computer program product comprising at least one non-transitory computer-readable medium including program instructions that, when executed by at least one processor, cause the at least one processor to:
receive, from a radio-frequency identification (RFID) reader, a plurality of identifiers read from a plurality of RFID tags on a plurality of medical devices in production line, wherein the plurality of medical devices in the production line are individually moved adjacent to an antenna of the RFID reader by a conveyor; and
for each identifier of the plurality of identifiers:
determine whether that identifier is included in a list of expected identifiers; and
in response to determining that that identifier is not included in the list of expected identifiers, transmit, to the conveyor, a signal to remove a medical device of the plurality of medical devices associated with that identifier from the production line.

22. The computer program product of claim 21, wherein, for each medical device of the plurality of medical devices in the production line:
at least one sensor detects when that medical device is in a predetermined location relative to the antenna of the RFID reader and transmits, in response to detecting that that medical device is in the predetermined location relative to the antenna of the RFID reader, a trigger signal to the RFID reader to trigger the RFID reader to attempt to read a RFID tag of the plurality of RFID tags on that medical device; and
the RFID reader attempts to read the RFID tag on that medical device in response to receiving the trigger signal from the at least one sensor, and
wherein the program instructions, when executed by the at least one processor, further cause the at least one processor to:
for each medical device of the plurality of medical devices in the production line, receive, from the RFID reader, at least one of: (i) an indication of whether a read attempt associated with that medical device failed to read an identifier associated with that medical device, (ii) the identifier associated with that medical device, or any combination thereof.

23. The computer program product of claim 22, wherein the program instructions, when executed by the at least one processor, further cause the at least one processor to:
in response to receiving an indication that the read attempt associated with that medical device failed, control the conveyor to remove that medical device from the production line.

24. The computer program product of claim 21, wherein the program instructions, when executed by the at least one processor, further cause the at least one processor to determine, for each identifier, whether that identifier is included in the list of expected identifiers by:
querying, based on that identifier, a database including the list of expected identifiers.

25. The computer program product of claim 24, wherein the program instructions, when executed by the at least one processor, cause the at least one processor to immediately query, for each identifier, the database including the list of expected identifiers in response to receiving that identifier from the RFID reader.

26. The computer program product of claim 24, wherein the plurality of medical devices in the production line are individually moved adjacent to the antenna of the RFID reader by the conveyor in a series of cycles, wherein each cycle of the series of cycles includes a subset of medical devices of the plurality of medical devices in the production line being individually moved adjacent to the antenna of the RFID reader by the conveyor, and wherein the program instructions, when executed by the at least one processor, further cause the at least one processor to:
for each cycle of the series of cycles:
after each medical device included in the subset of medical devices in that cycle is individually moved adjacent to the antenna of the RFID reader by the conveyor, query, based on a subset of identifiers associated with that subset of medical devices in that cycle, the database including the list of expected identifiers.

27. The computer program product of claim 24, wherein the program instructions, when executed by the at least one processor, further cause the at least one processor to:
obtain a batch number associated with the plurality of medical devices; and
control, based on the batch number, the database to synchronize the list of expected identifiers with a further database that includes a plurality of lists of expected identifiers associated with a plurality of batch numbers.

28. The computer program product of claim 21, wherein the plurality of medical devices includes a plurality of syringes.

29. The computer program product of claim 21, wherein the plurality of identifiers includes a plurality of electronic product codes.

30. The computer program product of claim 21, wherein the program instructions, when executed by the at least one processor, further cause the at least one processor to:
for each identifier of the plurality of identifiers:
in response to determining that that identifier is included in the list of expected identifiers, control the conveyor to maintain the medical device of the plurality of medical devices associated with that identifier in the production line.
